(19) Europäisches Patentamt
European Patent Office
Office européen des brevets

(11) Publication number: **0 121 138 B1**

(12) # EUROPEAN PATENT SPECIFICATION

(45) Date of publication of patent specification: **06.03.91**

(51) Int. Cl.⁵: **C12N 15/55**, C12N 15/70, C12N 9/86, //C12R1/07, C12R1/19

(21) Application number: **84102440.9**

(22) Date of filing: **07.03.84**

Divisional application 88121510.7 filed on 07/03/84.

The file contains technical information submitted after the application was filed and not included in this specification

(54) **Plasmids, methods for contruction of the same, microorganisms carrying the plasmids and methods for cultivation of the microorganisms.**

(30) Priority: **08.03.83 JP 38087/83**
**08.03.83 JP 38089/83**
**09.12.83 JP 232507/83**
**09.12.83 JP 232508/83**

(43) Date of publication of application:
**10.10.84 Bulletin 84/41**

(45) Publication of the grant of the patent:
**06.03.91 Bulletin 91/10**

(84) Designated Contracting States:
**AT BE CH DE FR GB IT LI NL SE**

(56) References cited:
**EP-A- 0 036 259**
**US-A- 4 411 994**

**AGRICULTURAL AND BIOLOGICAL CHEMISTRY, vol. 40, no. 5, May 1976 (Tokyo, JP); T. SUNAGA et al.:"Production of Penicillinase by an Alkalophilic Bacillus", pp. 1363-1367**

**FEBS LETTERS, Vol. 95, No. 1(1978), pp. 76-80**

(73) Proprietor: **RIKAGAKU KENKYUSHO**
**2-1 Hirosawa**
**Wako-shi Saitama-ken(JP)**

(72) Inventor: **Horikoshi, Koki**
**No. 4-39-8, Sakuradai**
**Nerima-ku Tokyo(JP)**
Inventor: **Kudo, Toshiaki**
**No. 1-21-20-606, Taira-cho**
**Meguro.ku Tokyo(JP)**
Inventor: **Kato, Chiaki**
**Nisshinjyutaku 1-307, No. 2-1607-2**
**Nisshin-cho Ohmiya-shi Saitama-ken(JP)**
Inventor: **Honda, Hiroshi**
**No. 1-1-1, Hinodai**
**Hino-shi Tokyo(JP)**

(74) Representative: **Vossius & Partner**
**Siebertstrasse 4 P.O. Box 86 07 67**
**W-8000 München 86(DE)**

**EP 0 121 138 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel plasmids, which are capable of inducing the extra cellular secretion of penicillinase in Escherichia coli to methods for the construction of said plasmids novel microorganisms carrying said plasmids, and for methods for the extracellular production of penicillinase comprising the cultivation of said microorganisms.

Plasmids are non-chromosomal elements of cyclic DNA found in a microorganism cell. Plasmids are currently being used as a means for recombination of microorganism genes and are becoming more and more important in the field of fermentation industry.

Studies have recently been done on plasmids carrying foreign DNA having genetic information of metabolic products of specific demand for growth of microorganism, as shown in production of amino acids or peptides. Some plasmids have been introduced into host microorganisms to obtain transformants. Methods have been proposed for producing relatively low molecular compounds such as amino acids and peptides by cultivating the transformants. However, the degree of propagation of plasmids carrying genes for production of high-molecular substances depends on the nature of host microorganisms and those plasmids have not effectively been expressed. Furthermore, no effective methods for cultivation of such transformants have been established.

It has not been possible to selectively obtain a certain extracellular high-molecular product by cultivating a microorganism transformed with a plasmid carrying foreign DNA fragment having genetic information of extracellular production of high-molecular substances which are metabolic products of other microorganisms. Such extracellular production has not successfully been done even when a transformant of Escherichia species, which is usually used as a host microorganism, is used.

US-A-4,411,994 of W.Gilbert et al discloses a process for producing specific proteins in bacteria and having them excreted from the bacterial cell. This process comprises inserting the DNA coding for the desired non-bacterial protein or part of a protein by recombinant techniques into a plasmid or phage gene which codes for either a periplasmic or an extracellular protein, hereinafter called a "carrier protein", then transforming a bacterial host with the recombinant DNA and culturing the transformed host to excrete the protein. The process of this patent provides a means for producing a selected protein by employing a gene for a carrier protein which has a leader sequence of hydrophobic amino acids at its amino terminus.

The cell wall of Escherichia coli which has often been used for production of useful physiologically active substances, consists of three kinds of membrane: inner membrane, peptido glycan and outer membrane. The space between the inner and outer membrane is called the periplasmic space. The process of US-A-4,411,994 has succeeded in the excretion of the products into the periplasmic space but not into the culture medium through the outer membrane or outside the bacterial host cell. In the present invention, by inserting the DNA having genetic information of extracellular production of high-molecular substances into the plasmid to obtain a hybrid plasmid and culturing the host transformed with the hybrid plasmid, it is possible to excrete useful physiologically active substances through the outer membrane and recover them directly from the culture medium. Thus, the present invention makes mass production of useful physiologically active substances possible, which has never been possible by the prior art processes.

Therefore, the technical problem underlying the present invention is to provide plasmids which are capable of inducing the extracellular secretion of penicillinase in E. Coli, methods for the construction of said plasmids, novel microorganisms carrying said plasmids and methods for the extra cellular production of penicillinase comprising the cultivation of said microorganisms.

The novel plasmids are constucted from a vector plasmid and a DNA fragment containing the genetic information for the extracellular production of penicillinase. These DNA fragments are obtained from microorganisms of the genus Bacillus ."Extracellular production" " means that the penicillinase produced is secreted by the cells into the culture medium.

These plasmids can be prepared by a process which comprises preparing, with a restriction enzyme, a chromosomal DNA fragment coding for extracellular production of penicillinase, digesting a plasmid vector DNA with a restriction enzyme which does not interfere with the genetic information carried on the chromosomal DNA fragment, treating the chromosonal DNA fragment and the digested vector plasmid with DNA ligase to form a recombinant plasmid and isolating the recombinant plasmid.

Novel microorganisms according to the present invention are those which belong to the genus Escherichia which contain the recombinant plasmid and which have the capability of extracellular production of penicillinase.

The present invention also provides a method for the extracellular production of penicillinase by bacteria comprises inoculating said microorganism into a culture medium containing an inorganic salt necessary for the microorganism to grow, or into a culture medium containing a selected carbon source together with the inorganic salt, keeping on culturing the microorganism after the concentration of the microorganism cells reached the maximum and until the production and accumulation of the high-molecular substances in the media reach the maximum. The figures show

Fig.1a shows bacterial growth and penicillinase production by Escherichia coli HB101(pEAP2) of the present invention.

Fig.1b shows percentages of extracellular, intracellular, and periplasmic cellular penicillinase production by Escherichia coli HB101(pEAP2).

Fig.2 shows bacterial growth and penicillinase production by Bacillus sp. No. 170.

Fig.3a shows bacterial growth and penicillinase production by Escherichia coli HB101(pBR322).

Fig.3b shows percentages of extracellular, intracellular, and periplasmic cellular penicillinase production by Escherichia coli HB101(pBR322).

Fig.4 shows the restriction enzyme cleavage map for the plasmid pEAP2.

The term "high molecular substances" used in the specification and the claims of this application means useful physiologically active compounds including bacterial metabolic products such as enzyme proteins, antibiotics and the like, and biologically active proteins of mammalian origin.

The present invention will now be explained in detail.

(a) The Plasmids and their Construction

The novel plasmids of the present invention are constructed by inserting a foreign DNA into a plasmid (extrachromosomal DNA or vector DNA), such as Col $E_1$, found in Escherichia cells.

The vector DNA which can be used in the present invention may have been isolated from natural sources.

The DNA fragments unnecessary for self-reproduction may have been deleted. Specific examples for vectors which may be used are the plasmids $ColE_1$, pMB9, pBR322, pSC101, R6K and lambda phage.

Foreign DNA fragments or exogenates which can be inserted into the vector DNA are the genes carrying the genetic information for the extracellular production or secretion of penicillinase.

Exogenates or DNA fragments which can be used in the present invention include the genes carrying the genetic information for the extracellular production or secretion of penicillinase. They are obtained from a microorganism which belongs to the genus Bacillus.

An example of a microorganism containing DNA which carries the genetic information for the extracellular production of penicillinase and which can be used in the present invention is Bacillus sp. No. 170 (Horikoshi K. et al (1976) Agric. Biol. Chem., 40 1363-1367).

For the purpose of the insertion of foreign DNA into a vector DNA, any conventional method can be used in the present invention. For instance, chromosomal DNA is digested with a suitable restriction enzyme or endonuclease to obtain a foreign DNA fragment or an exogenate which is then mixed with a vector DNA which has been treated with the same restriction enzyme, and the mixture is ligated by a suitable ligase. Thus, as described before, the novel plasmids of the present invention can be obtained by preparing, with a restriction enzyme, a DNA fragment coding for the extracellular production of penicillinase, digesting a plasmid vector DNA with a restriction enzyme which does not interfere with genetic information carried on the DNA fragment, treating the DNA fragment and the digested vector plasmid with DNA ligase to construct a recombinant plasmid and isolating the recombinant plasmid by a conventional manner [see Bolivar et. al., Gene, 2, 95 (1977)].

The novel plasmid pEAP2 can be constructed from a fragment of Bacillus sp. No . 170 chromosomal DNA and plasmid pMB9. The restriction enzyme map of plasmid pEAP2 is shown in Fig. 4. As seen from Fig. 4, plasmid pEAP2 is constructed from plasmid PMB9 into which the fragment of Bacillus sp. No. 170 chromosomal DNA coding for extracellular penicillinase production is inserted at the Hind III restriction site of plasmid pMB9. Thus, plasmid pEAP2 is a cyclic DNA molecule of about 7700 nucleotide base pairs (7.7 kb), consisting of pMB9 DNA and a fragment of Bacillus sp. No. 170 chromosomal DNA of about 2000 nucletide base pairs (2 kb).

(b) Preparation of Microorganisms

The recombinant plasmids constructed from the chromosomal DNA fragments and the vector DNA plasmids are introduced into a host microorganism of the genus Escherichia by a conventional transformation technique. Cultivation is kept on until a stabilized genotype is established in order to obtain a transformant carrying both genotypes on the selected chromosomal DNA and on the vector DNA. For this purpose, a conventional, so called shotgun method can be used.

A typical host microorganism which can be used in the present invention is Escherichia coli HB101 which is a hybrid strain of Escherichia coli K-12 and Escherichia coli B.

The transformant, Escherichia coli HB101 (pEAP2) which is prepared by introducing plasmid pEAP2 into Escherichia coli HB 101 has the same microbiological properties as the host, Escherichia coli HB101, except penicillin resistance [Molecular Cloning,A Laboratory Manual, p.504 (1982), Genotype: $F^-$ , hsdS20

($r^-_B$, $m_B$), recA13, ara-l4, proA2, lacYl, galK2, rpsL20(5m$^\gamma$), xyl-5, mt1-1, supE44λ]. E. coli HB101 (pEAP2) is further characterized by the property of plasmid pEAP2, that is, the capability of extracellular production of penicillinase.

The extracellular production of penicillinase by Escherichia coli HB101 (pEAP2), as shown in the Examples, reaches more than 80% of total production including the intracellular production and remains after long cultivation. By contrast, more than 80% of the total penicillinase production of the known strain Escherichia coli HB101 (pBR322) is intracellular and the extracellular penicillinase production of the known strain, Bacillus sp. No. 170 can not be kept for a long period of time.

The present invention which provides Escherichia coli HB 101 (pEAP2) having the capability of extracellular production of enzyme proteins such as penicillinase is not only novel but also inventive. Escherichia coli HB101 (pEAP2) can produce and secrete into the medium in addition to large amounts of penicillinase, large amounts of other enzyme proteins which can also be collected. More specifically, it has been discovered that Escherichia coli HB101 (pEAP2) produces and secretes into the medium from the cells large amounts of alkaline phosphatase,β-galactosidase and about ten kinds of proteins which have been observed only within the cells and never outside the cells.

Such extracellular production by Escherichia coli HB101 (pEAP2) demonstrates that the DNA of about 2000 nucleotide base pairs carried on plasmid pEAP2 provides the host with the capability of extracellular production of the metabolic products.

(c) Cultivation of the microorganisms

For culturing the transformants prepared in step (b), any culture media can be used which are suitable for the production of specific substances for which specific genetic information codes and which are suitable for growth of the microorganisms of the genus Escherichia. in the process of the present invention. It is necessary to culture the microorganism in a culture medium containing an inorganic salt necessary for the microorganism to grow, or a selected carbon source together with the inorganic salt, and to keep on culturing the microorganism, after the concentration of the microorganism cells reached the maximum, and until the production and accumulation of the high-molecular substances in the media reach the maximum.

Examples of the inorganic salt which can be used in the present invention include sodium and potassium salts such as sodium chloride, sodium sulfate, potassium chloride and the like, among which sodium chloride is preferred. The media containing the inorganic salt or the selected carbon source together with the inorganic salt can contain such carbon sources as glucose, sucrose, lactose, maltose, glycerol, bran, xylan and the like, such nitrogen sources as ammonia water, ammonium salts and the like, inorganic ions and optionally such nutrients as amino acids, vitamins and the like.

The culture media which can be used in the present invention are those which use, as a basic medium, LB-broth (containing tryptone, yeast extract and NaCl), BPB-broth (Difco Laboratories; containing polypeptone, yeast extract and $K_2HPO_4$) , nutrient broth (Difco 0001), tryptone-NaCl broth or the like.

Since more than 80% of total products remain in the intracellular fraction in a medium containing no inorganic salt, it is necessary to use a medium containing an inorganic salt so that most products are secreted into the culture medium of the cells. The amount of inorganic salt used is in the range of 0.5 - 3.0% by weight on the basis of the culture medium.

In the culture of Escherichia coli HB101 (pEAP2), excellent results are obtained by the use of LB-broth to which glucose and further glycerol have been added as carbon sources, preferably in an amount of 0.1 and 0.2% by weight on the basis of the culture medium, respectively.

Although culture conditions such as pH, temperature, oxygen supply or the like can be changed for the optimum growth of the microorganism of the genus Escherichia, it is necessary, in the present invention to keep on culturing the microorganism after the concentration of the microorganism cells reached the maximum, that is to say, after the late logarithmic phase and until the production and accumulation of the high-molecular substances in the medium reach the maximum.

After the inoculation of the microorganism of the genus Escherichia into the medium the cell concentration reaches the maximum after 5 to 20 hours and the production and accumulation of the high-molecular substances reach the maximum after 12 to 48 hours. Although the pH of the culture medium is not critical, it is preferably in the range of pH5 - pH8, most preferably pH7. Thus, without further addition of inorganic salt necessary for the microorganism to grow, carbon sources and the like during cultivation, the microorganism produces large amounts of extracellular high-molecular substances which can conveniently be collected.

According to the culture method of the present invention such high-molecular fermentation products as antibiotics, polysaccharides can be produced in a significant amount in addition to enzyme proteins. This method can be applied to the cultivation of any microorganisms transformed with a plasmid carrying a foreign DNA coding for biologically active high-molecular substances such as hormone peptides (e.g. insulin) or interferon and the DNA coding for extracellular production or secretion of biologically active high-molecular substances, which leads to mass production of the high-molecular substances such as insulin, interferon and the like.

As explaind above, the present invention contributes to the industrial production of useful high-molecular substances.

Examples of microorganisms which may be used in this invention include (i) Bacillus sp. No. 170, and Escherichia coli HB101 (pEAP2) which were deposited with the Fermentation Research Institute, Agency of Industrial Science and Technology, Ministry of International Trade and Industry, Japan, International Depository Authority (hereinafter referred to as "FERM") under the accession numbers, FERM BP-467 and FERM BP-468, respectively. They are on deposit with FERM in an unrestricted deposit permitting unrestricted access to the culture.

The depository and accession numbers of the above mentioned microorganisms are shown below:

| | Microorganims | Depository | Accession No. |
|---|---|---|---|
| (i) | Bacillus sp. No. 170 | FERM | FERM BP-467 |
| (ii) | Escherichia coli HB101 (pEAP2) | FERM | FERM BP-468 |

The applicant will maintain the deposition of FERM BP-467 and FERM BP-468 in unrestricted form until the end of the duration this patent, and thus said microorganism strains will be available to any third party at any time until the end of the duration of the present patent.

Methods for the construction of the plasmids of the present invention, methods for the preparation of the transformant Escherichia coli HB101 (pEAP2), and the extracellular production of penicillinase by this transformant will be explained hereinafter in the examples.

Example 1

(1) Preparation of a chromosomal DNA having genetic information for the production of penicillinase.

Alkalophilic Bacillus sp. No. 170 (FERM BP-467) having the capability of extracellular production of penicillinase was cultured at 30°C with shaking for 19 hours in the broth (containing 2.0g of glycerol, 5.0g of yeast extract, 5.0g of polypeptone, 10g of $K_2HPO_4$, 0.2g of $MgSO_4 \cdot 7H_2O$, 10g of $NaHCO_3$ in one liter of deionized water; pH9.0). The cells in the latter logarithmic phase were collected. The chromosomal DNA was extracted by the phenol extraction method and purified to obtain 5mg of the chromosomal DNA.

(2) Insertion of chromosomal DNA fragment into vector DNA.

The chromosomal DNA (10μg) obtained in step (1) was digested with the restriction enzyme Hind III at 37°C for 5, 10, 20, 30 and 60 minutes to obtain DNA fragments.

Plasmid pMB9 [Bethesda Research Laboratories, U.S.A., tetracyclin resistant ($Tet^r$)] used as the vector was cut with Hind III, heated at 65°C for 5 minutes, and then mixed with the DNA fragments. The mixture was treated with $T_4$ phage DNA ligase at 10°C for 24 hours and then heated at 65°C for 5 minutes.

Two times volume of ethanol was added to the mixture. Plasmids carrying the chromosomal DNA fragments were precipitated and collected.

(3) Transformation of the microorganism with plasmids having gene for extracellular production of penicillinase.

Escherichia coli HB101 which is a hybrid strain of Escherichia coli K-12 and Escherichia coli B, was inoculated in 10ml of LB-broth [containing 10g of tryptone (Difco Laboratories, Detroit, Mich.), 5g of yeast extract, 1g of glucose and 10g of NaCl in one liter of deionized water; pH was adjusted to 7.0] and cultured at 37°C with shaking until the latter logarithmic phase. The cells were collected and suspended in an ice-cold $CaCl_2$ solution of 0.03M (final concentration) to obtain competent cells. The cell suspension and the plasmid solution obtained in step (2) were combined and kept on ice for 60 minutes: The mixture was heated to 42°C for 1 to 2 minutes to introduce the plasmid DNA into the cells. This cell suspension was inoculated in fresh LB-broth and cultured at 37°C for 3 to 5 hours with shaking. The cells were collected and washed to obtain Escherichia coli HB101 (pEAP2) having the capability of penicillinase production.

Example 2

Perparation and purification of plasmid pEAP2 1. Culture growth of bacterial cells using Escherichia coli HB101 (pEAP2) [FERM BP-468]

Preculture in LB. Over night at 37°C.
↓
Culture in M-9. Add chloramphenicol after 130 Klett units. 16hr at 37°C.
↓
Centrifugation. 5000rpm,20min at 4°C.
↓
Cell pellets. Stored at -80°C.

2. Purification of plasmid DNA.

Cell pellets
↓ 10ml, 20%Sucrose, 50mMTris, 1mMEDTA PH8. Suspended, Keep on ice.
50ml polypropylene centrifuge tube.
↓ 2ml, 0.25M EDTA. 1ml, Lysozyme (5mg/ml of 0.025M Tris, PH8.) 0.1ml, RNases (10mg/ml)
Cell lysis. Mix gently. Stand 15 to 30 min on ice.
↓ 5ml, 3x Triton. Mix gently. Stand 15 to 45 min on ice.
Centrifugation 17,000rpm, 40min at 4°C.
↓
Supernatant soln in plastic cylinder.
↓
250ml glass bottle.
↓ 2/3 vol DD $H_2O$ 2/3 vol Cold saturated phenol. Mix gently
Centrifugation. 6500rpm, 15min at 4°C.
↓
Upper phase.
↓ Equal vol. of phenol; Chloroform.
Centrifugation. 6500rpm, 15min at 4°C.

↓
Upper phase in 250ml bottle.
  1/25 vol 5M NaCl.
  2 vol EtOH at -20°C.
    Over night at -20°C.
↓
Centrifugation. 6500rpm, 60min at -20°C.
↓
DNA pellet. Dry excess liquid.
  5ml A-50 buffer, resuspended.
  1ml sterile 80% glycerol, mix gentry.
↓
A-50 column. (2x35cm,1fraction=4ml.)
↓
DNA fraction. ($A_{260}$ peak.)
  2 vol EtOH at -20°C. Over night at -20°C.
↓
Centrifugation. 6500rpm, 60min at -20°C.
↓
DNA pellet.
  2.1ml TEN buffer in 5ml cellulose nitrate tube.
  2.2g CsCl, mix.

  (In dark.)

  150ul PdI(2mg/ml). Mix well.
  2ml mineral oil on top of tube.
↓
CsCl gradient centrifugation. 36,000rpm,40hr at 20°C.
  Visible with UV.
    Upper band; chromosomal & nicked DNA.
    Lower band; covalently closed p-DNA.
↓
Collect of lower band DNA by drop wise.
↓
Dowex 50W-X8 column. UV check.
  (In light.)
↓
Dialysis against 2 to 4l of 10mM Tris, 1mM EDTA pH8.
  Over night at 4°C.
↓
In 30ml cortex centrifuge tube. vol.
  1/25 vol 5M NaCl.
  2 vol EtOH at -20°C.
    Over night at -20°C.
↓
Centrifugation. 6500rpm,60min at -20°C.
↓
DNA precipitates.
  1-2ml TEN buffer.
↓
Purified plasmid DNA (1mg/1-broth) Stored at -20 to 70°C.

Example 3

Escherichia coli HB101 (pEAP2) (FERM BP-468) obtained in step (3) of Example 1 was inoculated in 500ml-flasks containing 100ml of LB-broth (containing 10g of tryptone, 5g of yeast extract, lg of glucose, 2g of glycerol, and 10g of NaCl in one liter of water) and cultured at 37° C with shaking. Cell growth (cell amount) was measured by optical density at 660nm.

Extracellular and intracellular penicillinase activities were assayed by the modified Sargent's method [Sawai et al; Antimicrob. Agents Chemother. 13, 910 (1978)], wherein the enzyme quantity hydrolysing at 30° C one micromole of benzylpenicillin per one minute constitutes one unit of penicillinase.

As shown in Fig. 1, cell growth of the transformant reached the maximum after 16 hours cultivation and extracellular penicillinase activity began to increase after 20 hours and reached the maximum (about 20 units/ml) after 28 hours cultivation.

Extracellular penicillinase produced was very stable and, as shown in Fig. 1 a, the production was kept high even after 48 hours cultivation and reached more than 80% of total enzyme production. In contrast, intracellular penicillinase production was detected at an early phase (after 8 to 20 hours cultivation). But, the maximum was only 8 units/ml or about 10% of total enzyme production and furthermore, the intracellular production decreased quickly. No intracellular penicillinase activity was observed after 48 hours cultivation. Fig. 1 b shows percentages of extracellular and intracellular penicillinase activities on total enzyme activity. As a comparison, Bacillus sp. No. 170 (FERN BP-467) which is a DNA donor and Escherichia coli HB101 (pBR322) (carrying plasmid pBR322 having genetic information of penicillinase production) [Boyer et al; Gene, vol. 2, p.95-113 (1977)] were cultured and penicillinase activities were assayed.

Bacillus sp. No. 170 was inoculated in 500ml-flasks containing 100ml of the broth (containing 2.0g of glucose or glycerol, 5.0g of yeast extract, 5.0g of polypeptone, 1.0g of $K_2HPO_4$, 0.2g of $MgSO_4 \cdot 7H_2O$ and 10g of $NaHCO_3$ in one liter of water and adjusted to pH 9.0) and cultured at 37° C with shaking. Extracellular penicillinase activity in the culture fluid was observed every four hours. It reached the maximum (19 units/ml) after 12 hours cultivation and then quickly decreased. No extracellular penicillinase activity was observed after 40 hours cultivation (Fig. 2).

On the other hand, Escherichia coli HB101 (pBR322) was inoculated in 500ml-flasks containing 100ml of LB-broth which is the same as that used in the cultivation of Escherichia coli HB101 (pEAP2) and cultured at 37° C with shaking. As shown in Fig 3 a, intracellular penicillinase activity reached the maximum (35 units/ml) after 20 hours cultivation and it was more than 80% of total enzyme activity. But it decreased quickly. Extracellular penicillinase activity was less than 10% of total enzyme activity. Fig. 3 b shows percentages of extracellular and intracellular penicillinase activities on total enzyme activity.

Identification of Penicillinase:

The culture fluid of the transformant obtained in step (3) of Example 1 was centrifuged at 10,000 x g for 10 minutes. The supernatant fluid was brought to 80% saturation with ammonium sulfate. The precipitate was dissolved in water and the solution was dialyzed overnight against 0.05M phosphate buffer containing 0.1M NaCl (pH 7.0). The dialysate was applied on a Sephadex G-75 column equilibrated with the phosphate buffer to obtain purified penicillinase.

Similarly, the culture fluid of Bacillus sp. No. 170 was treated to obtain purified penicillinase.

For the identification of Escherichia coli HB101 (pEAP2) penicillinase with Bacillus sp. No. 170 penicillinase, effects of pH on activity, thermal stability and molecular weight of each penicillinase were investigated. As a result, the enzymes were identical with each other as shown below.

(a) Stability of the enzyme was investigated in buffer solutions of various pH values. The mixture was incubated at 30° C for 45 minutes.

Both the enzymes were stable between pH 7 and pH 9 and the optimum pH values for enzyme action were 6.0 to 7.0.

(b) Thermal stability was investigated as follows. The enzymes were dissolved in 0.05M phosphate buffer of pH 7.0 and the solutions were heated at the selected temperature for 10 minutes. The residual activities were measured at pH 7.0. Both the enzymes were stable up to 50° C.

(c) Estimation of molecular weight of the enzymes was done by using the Sephadex gel filtration method. Estimated molecular weight of both the enzymes was 27,000 to 22,000.

Example 4

Culture conditions for penicillinase production by Escherichia coli HB101 (pEAP2) (FERN BP-468) were examined in the various media. The other culture conditions were the same as those in Example 3.

Table 1

| Media | Time (hrs.) | Penicillinase Activity (U/ml) | | |
|---|---|---|---|---|
| | | Extracellular | Intracellular | Total |
| LB-broth | 20 | 14.7 (88.5%) | 1.9 (11.5%) | 16.6 |
| | 30 | 15.6 (84.8%) | 2.8 (15.2%) | 18.4 |
| BPB-broth | 20 | 1.6 (10%) | 14.6 (90%) | 16.2 |
| | 30 | 9.1 (39%) | 14.2 (61%) | 23.3 |

Effects of components of LB-broth on penicillinase production are given in Table 2. The concentration of tryptone, tryptose, polypeptone and NaCl was 1% by weight.

Table 2

| Component | Time (hrs.) | Penicillinase Activity (U/ml) Extracellular | Intracellular | Total |
|---|---|---|---|---|
| Tryptone NaCl | 20 | 14.7 (88.5%) | 1.9 (11.5%) | 16.6 |
| | 30 | 15.6 (84.8%) | 2.8 (15.2%) | 18.4 |
| Tryptone | 20 | 2.2 (20%) | 8.8 (80%) | 11.0 |
| | 30 | 3.1 (28.7%) | 7.7 (71.3%) | 10.8 |
| Tryptose NaCl | 20 | 13.7 (92.5%) | 1.1 (7.5%) | 14.8 |
| | 30 | 15.4 (90.5%) | 1.6 (9.5%) | 17.0 |
| Trytose | 20 | 1.4 (15.7%) | 7.5 (84.3%) | 8.9 |
| | 30 | 2.3 (20.9%) | 8.7 (79.1%) | 11.0 |
| Polypeptone NaCl | 20 | 16.4 (91.6%) | 1.5 (8.4%) | 17.9 |
| | 30 | 17.1 (95.5%) | 0.7 (4.5%) | 17.8 |
| Polypeptone | 20 | 1.8 (24%) | 5.7 (76%) | 7.5 |
| | 30 | 3.4 (36.9%) | 5.8 (63.1%) | 9.2 |

Effects of carbon sources of LB-broth on penicillinase production are given in Table 3. The concentration of glucose, starch, sucrose and maltose was 0.1%, that of glycerol was 0.2% and that of NaCl was 1%.

Table 3

| Carbon source | Time (hrs.) | Penicillinase Activity (U/ml) | | |
|---|---|---|---|---|
| | | Extracellular | Intracellular | Total |
| Glucose NaCl | 20 | 17.3 (94.0%) | 1.1 (6%) | 18.4 |
| | 30 | 15.1 (96.7%) | 0.5 (3.3%) | 15.6 |
| Glycerol NaCl | 20 | 5.4 (90.0%) | 0.6 (10.0%) | 6.0 |
| | 30 | 5.2 (74.3%) | 1.8 (25.7%) | 7.0 |
| Starch NaCl | 20 | 6.6 (74.2%) | 2.3 (25.8%) | 8.9 |
| | 30 | 4.9 (55.7%) | 3.9 (44.3%) | 8.8 |
| Sucrose NaCl | 20 | 7.1 (78.0%) | 2.0 (22.0%) | 9.1 |
| | 30 | 6.5 (60.2%) | 4.3 (39.8%) | 10.8 |
| Maltose NaCl | 20 | 12.8 (89.3%) | 1.5 (10.7%) | 14.3 |
| | 30 | 11.8 (81.9%) | 2.6 (18.1%) | 14.4 |
| Glucose Glycerol NaCl | 30 | 20.0 (94.3%) | 1.2 (5.7%) | 21.2 |

Effects of NaCl on penicillinase production were examined using the media containing 1% of tryptone, 0.1% of glucose, 0.2% of glycerol, 1% of yeast extract and the indicated amount of NaCl . Results are given in Table 4.

Table 4

| Nacl (%) | Extracellular activity (U/ml) |
|---|---|
| 0 | 0.2 |
| 0.5 | 15.0 |
| 1.0 | 20.0 |
| 2.0 | 18.0 |
| 5.0 | 8.0 |
| 10.0 | 0.1 |

Example 5

Escherichia coli HB101, Escherichia coli HB101 (pMB9) and Escherichia coli HB101 (pEAP2) (FERN BP-468) were cultured in the same LB-broth as used in Example 3 at 37°C for 20 hours with shaking. Enzyme activities of alkaline phosphatase and β-galactosidase were measured by optical density at 420nm. Results are given in Table 5.

Table 5

| Micro-organism | Products | Activity (U/ml) | | |
|---|---|---|---|---|
| | | Extracellular | Intracellular | Total |
| | Proteins* | 0.04 (4%) | 1.04 (96%) | 1.08 (100%) |
| E. coli HB101 | Alkaline phosphatase | 0.02 (2%) | 1.31 (98%) | 1.33 (100%) |
| | β-Galactosidase | 0.03 (2%) | 1.20 (98%) | 1.23 (100%) |

| | | | | |
|---|---|---|---|---|
| | Proteins* | 0.01 (1%) | 0.78 (99%) | 0.79 (100%) |
| E. coli HB101 (pMB9) | Alkaline phosphatase | 0.01 (2%) | 0.47 (98%) | 0.48 (100%) |
| | β-Galactosidase | 0.00 (0%) | 0.80 (100%) | 0.80 (100%) |
| | Proteins* | 0.18 (21%) | 0.67 (79%) | 0.85 (100%) |
| E. coli HB101 (pEAP2) | Alkaline phosphatase | 0.29 (58%) | 0.21 (42%) | 0.50 (100%) |
| | β-Galactosidase | 0.09 (10%) | 0.81 (90%) | 0.90 (100%) |
| | Penicillinase | 10.70 (83%) | 2.20 (17%) | 12.90 (100%) |

* Proteins contain about 10 kinds of proteins and are represented in mg/ml.

## Claims

1. A recombinant plasmid which is capable of inducing the extracellular secretion of penicillinase in Escherichia coli transformed with said plasmid, said plasmid comprising:
   (a) a restriction fragment of the chromosomal DNA of Bacillus 5p. No. 170 (FERM BP-467) prepared with a first restriction enzyme, preferably a HindIII fragment having a length of about 4kb, which codes for the extracellular secretion of penicillinase; and
   (b) a vector plasmid DNA fragment, preferably a DNA fragment of pMB9, prepared with a second restriction enzyme which does not interfere with the genetic information carried on the restriction fragment of (a).

2. The recombinant plasmid according to claim 1, wherein the vector plasmid is plasmid pMB9.

3. The recombinant plasmid according to claim 1, which is the plasmid pEAP2 (FERM BP-468).

4. A host organism of the genus Escherichia, preferably of the species Escherichia coli, which is transformed with a recombinant plasmid according to any one of Claims 1 to 3.

5. A host organism according to Claim 4, which is the strain Escherichia coli HB101 and which is capable of extracellular secretion of penicillinase.

6. A method for the construction of a recombinant plasmid according to Claim 1, which comprises the steps of:
   (a) preparing with a first restriction enzyme a chromosomal DNA fragment of Bacillus sp. No. 170 (FEB BP-467) which codes for the extracellular secretion of penicillinase;
   (b) digesting a vector plasmid DNA with a second restriction enzyme which does not interfere with the genetic information carried on the chromosomal DNA fragment;

(c) treating said chromosomal DNA fragment and said digested vector plasmid DNA with DNA ligase to form the recombinant plasmid DNA; and
(d) isolating the recombinant plasmid DNA from a transformed host cell which exhibits a Penicillinase-producing phenotype.

**7.** The method according to Claim 6, wherein the first and second restriction enzyme is the restriction enzyme Hind III.

**8.** The method according to Claim 6, wherein the hector plasmid is pMB9.

**9.** The method according to claim 6, wherein the recombinant plasmid is plasmid pEAP2 (FERM BP-468).

**10.** A method for the extracellular production of penicillinase in which a transformant according to Claim 4 or 5 is cultivated and which comprises:
(a) inocculating a medium containing an inorganic salt which is a sodium salt or a potassium salt with the transformant;
(b) keeping on culturing the transformant after the concentration of the cells reached the maximum and until the production and accumulation of penicillinase in the medium reached the maximum.

**11.** The method of Claim 10, wherein the inorganic salt is sodium chloride or potassium chloride.

**12.** The method of Claim 10 or 11, wherein the inorganic salt is used in an amount of 0.5 to 3.0 % by weight of the medium.

**13.** The method of any one of Claims 10 to 12, wherein the transformant is cultured for 12 to 48 hours.

**Revendications**

**1.** Plasmide recombinant qui est capable d'induire la sécrétion extracellulaire de pénicillinase dans Escherichia coli transformé avec ledit plasmide, ledit plasmide comprenant:
(a) un fragment de restriction de l'ADN chromosomal de Bacillus sp. N° 170 (FERM BP-467) préparé avec une première enzyme de restriction, de préférence un fragment Hind III, ayant une longueur d'environ 4kb, qui code pour la sécrétion extracellulaire de la pénicillinase ; et
(b) un fragment d'ADN d'un plasmide vecteur, de préférence un fragment d'ADN de pMB9, préparé avec une seconde enzyme de restriction qui n'interfère pas avec l'information génétique portée par le fragment de restriction de (a).

**2.** Plasmide recombinant selon la revendication 1, dans lequel le plasmide vecteur est le plasmide pMB9.

**3.** Plasmide recombinant selon la revendication 1, qui est le plasmide pEAP2 (FERM BP-468).

**4.** Organisme hôte du genre Escherichia, de préférence de l'espèce Escherichia coli, qui est transformé avec un plasmide recombinant selon l'une quelconque des revendications 1 à 3.

**5.** Organisme hôte selon la revendication 4, qui est la souche Escherichia coli HB101 et qui est capable d'induire la sécrétion extracellulaire de pénicillinase.

**6.** Procédé de construction d'un plasmide recombinant selon la revendication 1, qui comprend les étapes consistant à:
(a) préparer, à l'aide d'une première enzyme de restriction, un fragment d'ADN chromosomal de Bacillus Sp. N° 170 (FERM BP-467) qui code pour la sécrétion extracellulaire de la pénicillinase;
(b) digérer l'ADN d'un plasmide vecteur à l'aide d'une seconde enzyme de restriction qui n'interfère pas avec l'information génétique portée par le fragment d'ADN chromosomal ;
(c) traiter ledit fragment d'ADN chromosomal et ledit ADN de plasmide vecteur digéré, avec l'ADN-ligase pour former un ADN de plasmide recombinant; et
(d) isoler l'ADN de plasmide recombinant à partir d'une cellule hôte transformée qui présente un phénotype producteur de pénicillinase.

7. Procédé selon la revendication 6, dans lequel les première et seconde enzymes de restriction sont des enzymes de restriction Hind III.

8. Procédé selon la revendication 6, dans lequel le plasmide vecteur est le pMB9.

9. Procédé selon la revendication 6, dans lequel le plasmide recombinant est le plasmide pEAP2 (FERM BP-468).

10. Procédé pour la production extracellulaire de pénicillinase, dans lequel un transformant selon la revendication 4 ou 5 est cultivé, et qui consiste à:
(a) inoculer le transformant dans un milieu contenant un sel minéral qui est un sel de sodium ou un sel de potassium;
(b) maintenir sous culture le transformant après que la concentration des cellules ait atteint le maximum et jusqu'à ce que la production et l'accumulation de pénicillinase dans le milieu ait atteint le maximum.

11. Procédé selon la revendication 10, dans lequel le sel minéral est le chlorure de sodium ou le chlorure de potassium.

12. Procédé selon les revendications 10 ou 11, dans lequel le sel minéral est utilisé en une proportion de 0,5 à 3,0 % en poids du milieu.

13. Procédé selon l'une quelconque des revendications 10 à 12, dans lequel le transformant est cultivé pendant 12 à 48 heures.

## Ansprüche

1. Rekombinantes Plasmid, das zur Induktion der extrazellulären Sekretion von Penicillinase in mit diesem Plasmid transformierten Escherichia coli fähig ist, enthaltend:
(a) ein mit einem ersten Restriktionsenzym hergestelltes Restriktionsfragment der chromosomalen DNA von Bacillus sp. Nr. 170 (FERM BP-467), vorzugsweise ein HindIII-Fragment einer Länge von etwa 4kb, das für die extrazelluläre Sekretion von Penicillinase Codiert; und
(b) ein mit einem zweiten Restriktionsenzym, das nicht mit der vom Restriktionsfragment (a) getragenen genetischen Information interferiert, hergestelltes Vektor-Plasmid-DNA-Fragment, vorzugsweise ein pMB9-DNA-Fragment.

2. Rekombinantes Plasmid nach Anspruch 1, wobei das Vektorplasmid das Plasmid pMB9 ist.

3. Rekombinantes Plasmid nach Anspruch 1, das das Plasmid pEAP2 (FERM BP-468) ist.

4. Wirtsorganismus der Gattung Escherichia, vorzugsweise der Art Escherichia coli, der mit einem rekombinanten Plasmid nach einem der Ansprüche 1 bis 3 transformiert ist.

5. Wirtsorganismus nach Anspruch 4, der der Stamm Escherichia coli HB101 ist und zur extrazellulären Sekretion von Penicillinase fähig ist.

6. Verfahren zur Herstellung eines rekombinanten Plasmids nach Anspruch 1, bei dem man die folgenden Schritte durchführt:
(a) Herstellung eines chromosomalen DNA-Fragments von Bacillus sp. Nr. 170 (FERM BP-467), das für die extrazelluläre Sekrektion von Penicillinase codiert, mit einem ersten Restriktionsenzym;
(b) Spaltung einer Vektor-Plasmid-DNA mit einem zweiten Restriktionsenzym, das nicht mit der vom chromosomalen DNA-Fragment getragenen genetischen Information interferiert;
(c) Behandlung des chromosomalen DNA-Fragments und der gespaltenen Vektor-Plasmid-DNA mit DNA-Ligase zur Bildung der rekombinanten Plasmid-DNA; und
(d) Isolierung der rekombinanten Plasmid-DNA aus einer transformierten Wirtszelle, die einen Penicillinasebildenden Phänotyp aufweist.

7. Verfahren nach Anspruch 6, bei dem das erste und zweite Restriktionsenzym das Restriktionsenzym HindIII ist.

8. Verfahren nach Anspruch 6, wobei das Vektorplasmid pMB9 ist.

9. Verfahren nach Anspruch 6, wobei das rekombinante Plasmid das Plasmid pEAP2 (FERM BP-468) ist.

10. Verfahren zur extrazellulären Herstellung von Penicillinase, bei dem eine Transformante nach Anspruch 4 oder 5 gezüchtet wird und bei dem man:
(a) ein ein anorganisches Salz, das ein Natriumsalz oder ein Kaliumsalz ist, enthaltendes Medium mit der Transformante beimpft;
(b) die Transformante, nachdem die Konzentration der Zellen das Maximum erreicht hat, weiter züchtet, bis die Bildung und Ansammlung der Penicillinase im Medium das Maximum erreicht hat.

11. Verfahren nach Anspruch 10, bei dem das anorganische Salz Natriumchlorid oder Kaliumchlorid ist.

12. Verfahren nach Anspruch 10 oder 11, bei dem das anorganische Salz in einer Menge von 0,5 bis 3,0 Gew.-% des Mediums verwendet wird.

13. Verfahren nach einem der Ansprüche 10 bis 12, bei dem die Transformante I2 bis 48 Stunden gezüchtet wird.

FIG. 1a

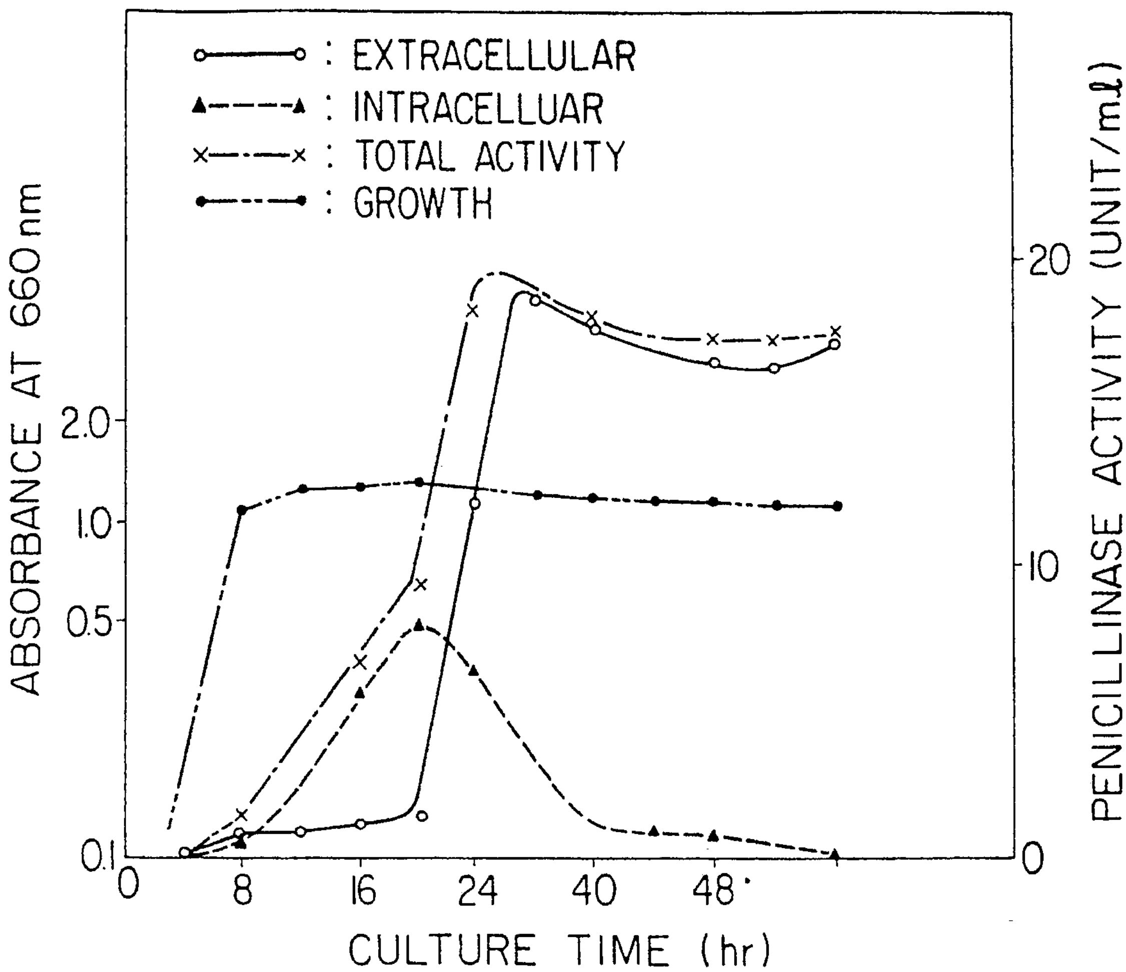
: EXTRACELLULAR
: INTRACELLUAR
: TOTAL ACTIVITY
: GROWTH
ABSORBANCE AT 660 nm
2.0
1.0
0.5
0.1
PENICILLINASE ACTIVITY (UNIT/ml)
20
10
0
8
16
24
40
48
CULTURE TIME (hr)

FIG. 1b

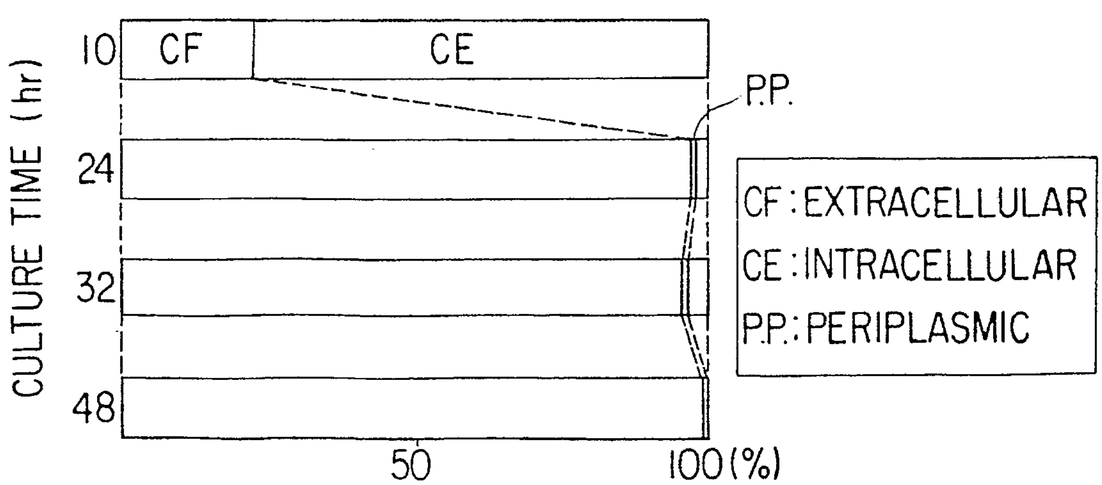
CULTURE TIME (hr)
10
24
32
48
CF
CE
P.P.
50
100(%)
CF: EXTRACELLULAR
CE: INTRACELLULAR
P.P.: PERIPLASMIC

FIG. 2

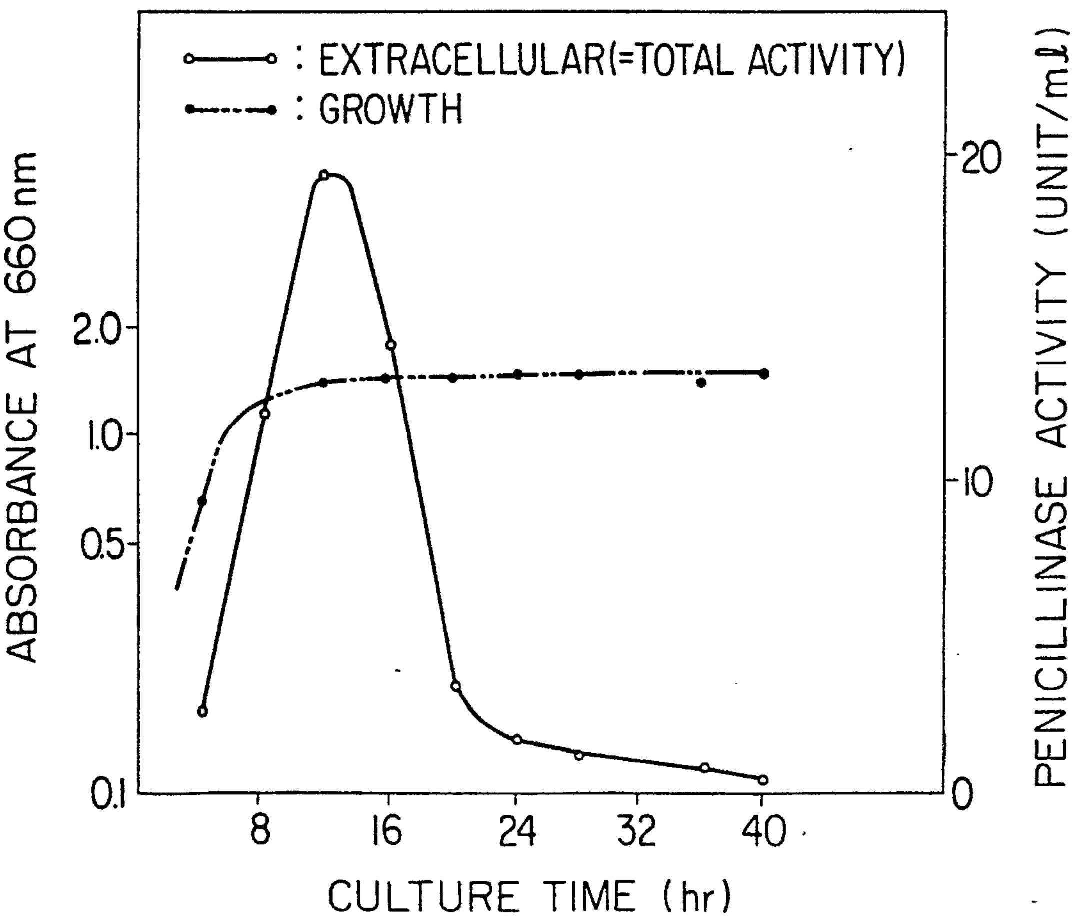
: EXTRACELLULAR(=TOTAL ACTIVITY)
: GROWTH
ABSORBANCE AT 660 nm
2.0
1.0
0.5
0.1
PENICILLINASE ACTIVITY (UNIT/ml)
20
10
0
8
16
24
32
40
CULTURE TIME (hr)

FIG. 3a

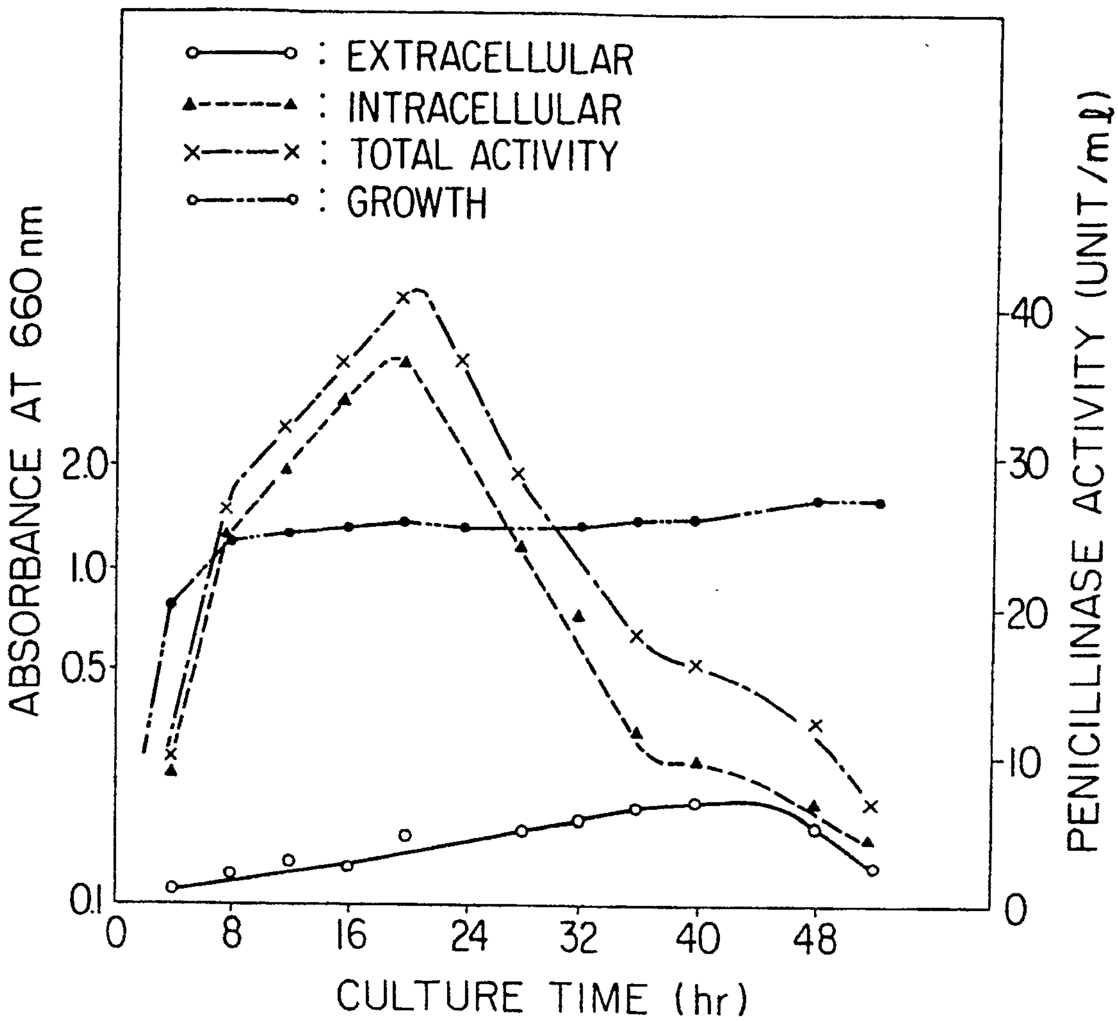
: EXTRACELLULAR
: INTRACELLULAR
: TOTAL ACTIVITY
: GROWTH
ABSORBANCE AT 660 nm
PENICILLINASE ACTIVITY (UNIT/mℓ)
2.0
1.0
0.5
0.1
40
30
20
10
0
0
8
16
24
32
40
48
CULTURE TIME (hr)

FIG. 3b

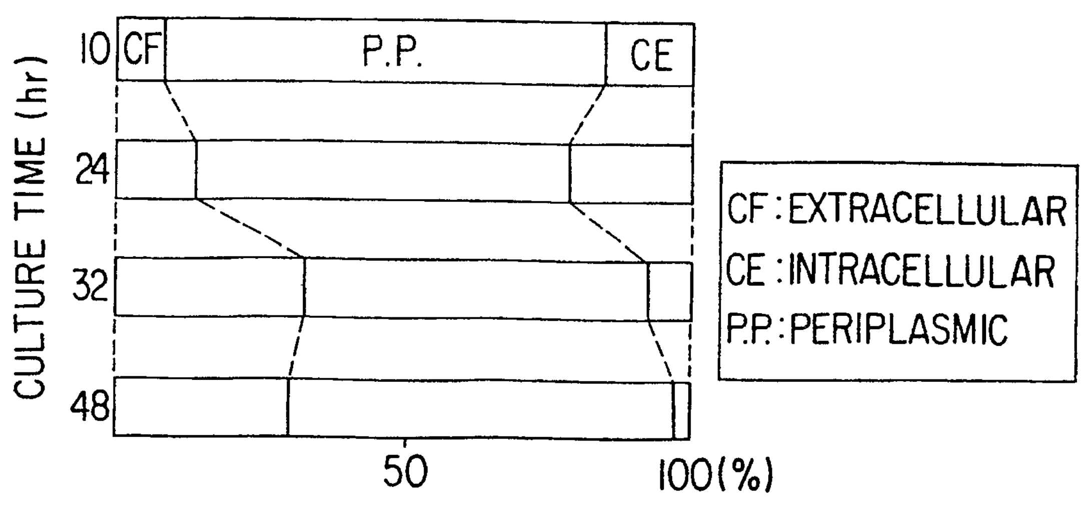
CULTURE TIME (hr)
10
24
32
48
CF
P.P.
CE
50
100(%)
CF: EXTRACELLULAR
CE: INTRACELLULAR
P.P.: PERIPLASMIC

# FIG. 4

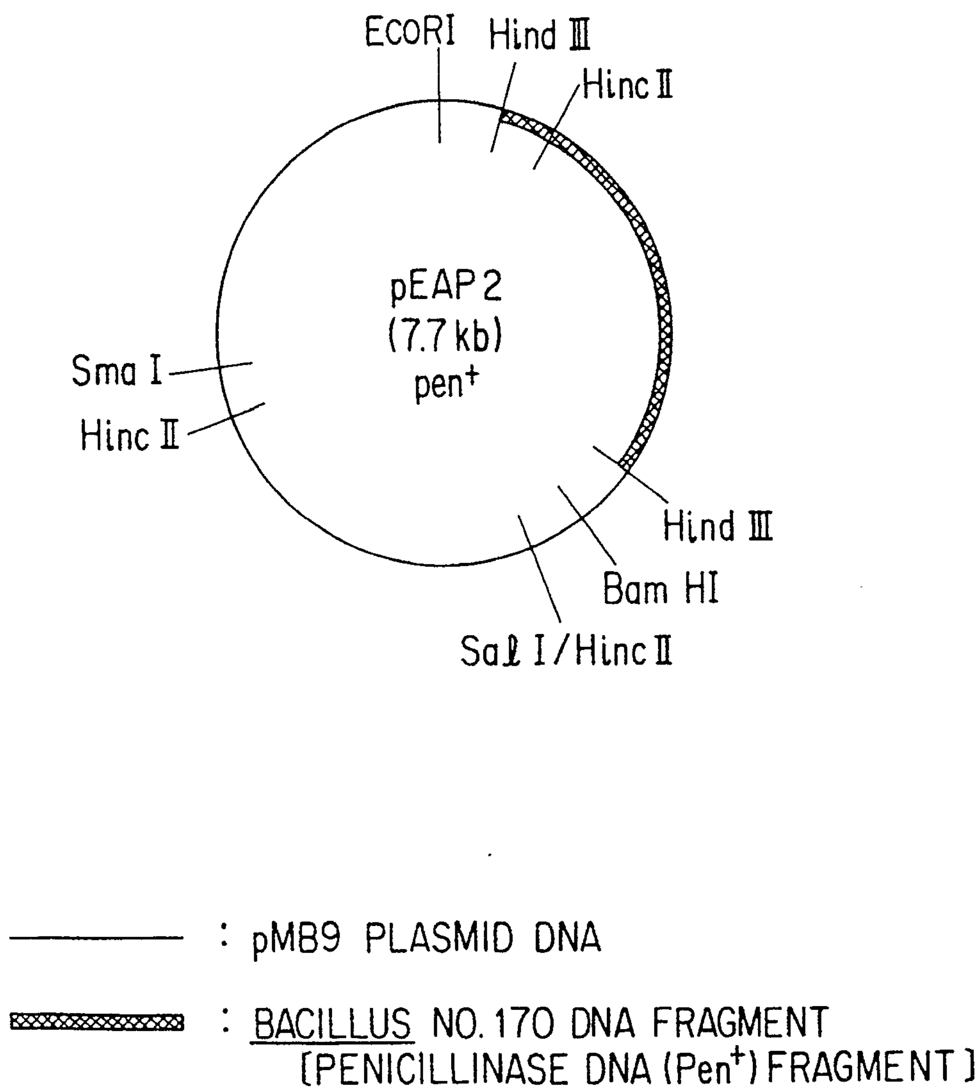
EcoRI
Hind III
Hinc II
pEAP2
(7.7 kb)
pen+
Sma I
Hinc II
Hind III
Bam HI
Sal I/Hinc II
: pMB9 PLASMID DNA
: BACILLUS NO. 170 DNA FRAGMENT
[PENICILLINASE DNA (Pen+) FRAGMENT]

FIG. 5

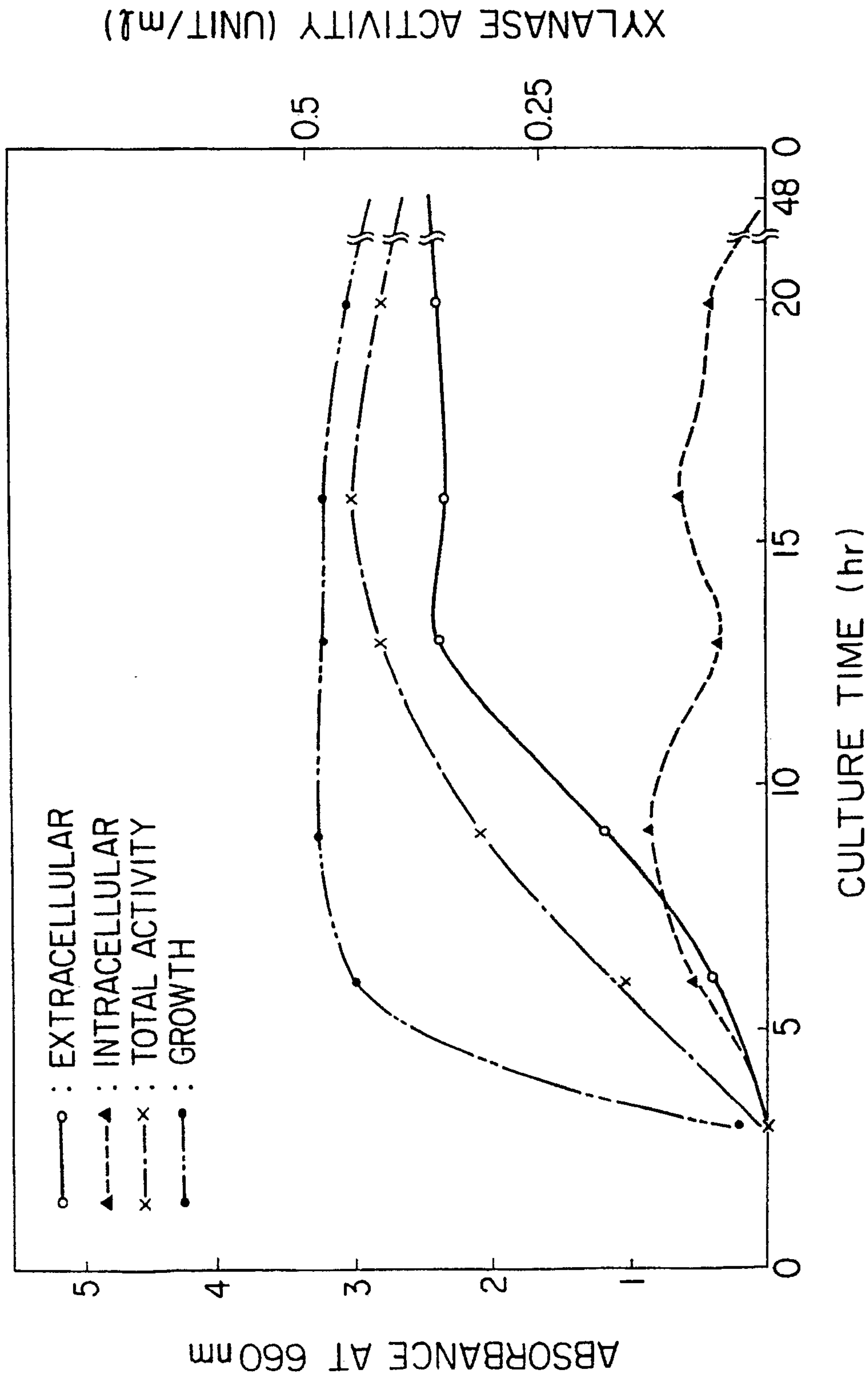
EXTRACELLULAR
INTRACELLULAR
TOTAL ACTIVITY
GROWTH
XYLANASE ACTIVITY (UNIT/mℓ)
0.5
0.25
0
ABSORBANCE AT 660 nm
5
4
3
2
1
0
5
10
15
20
48
CULTURE TIME (hr)

FIG. 6

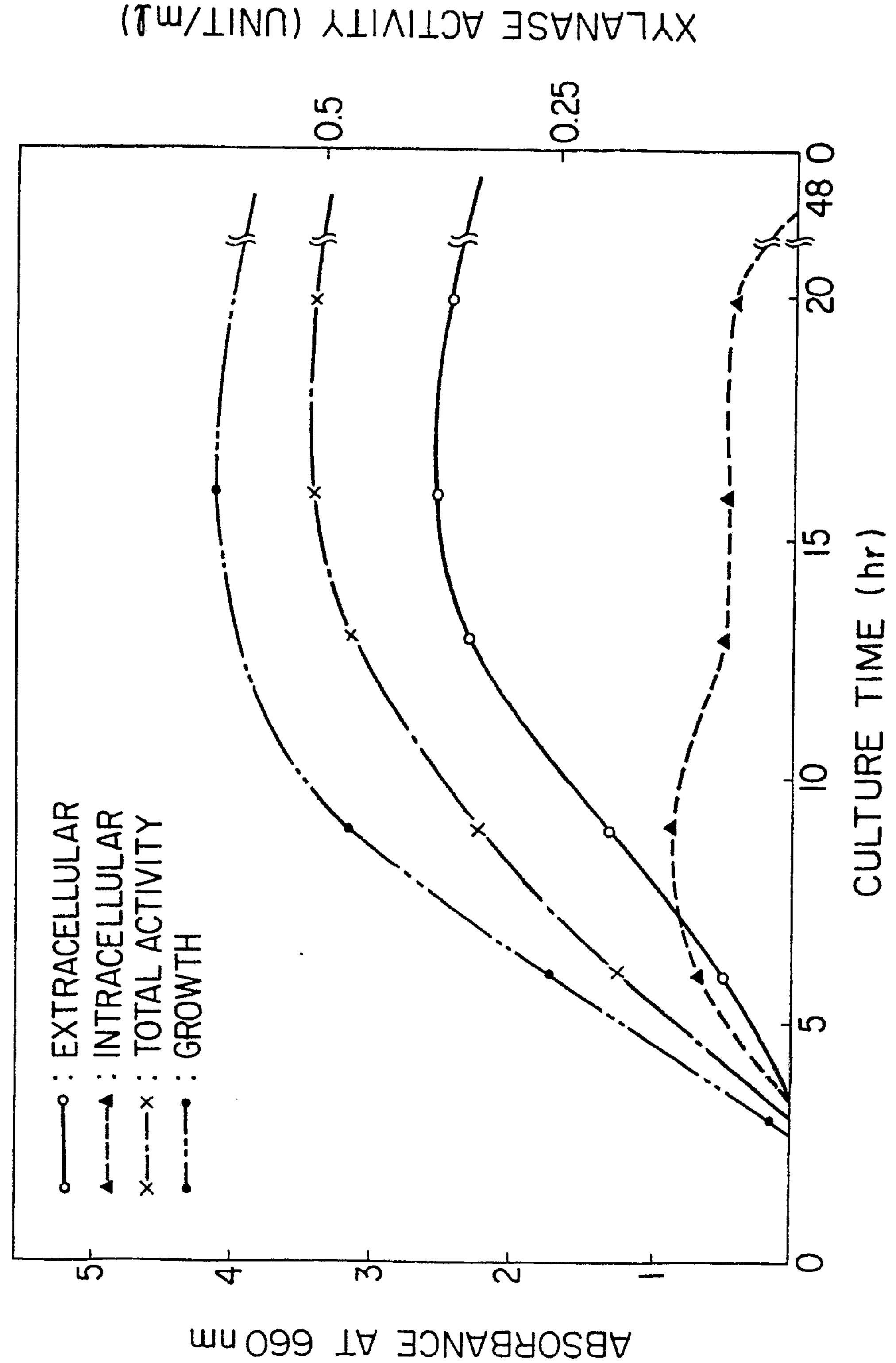

: EXTRACELLULAR
: INTRACELLULAR
: TOTAL ACTIVITY
: GROWTH
XYLANASE ACTIVITY (UNIT/mℓ)
0.5
0.25
0
ABSORBANCE AT 660 nm
5
4
3
2
1
0
CULTURE TIME (hr)
5
10
15
20
48

FIG. 7

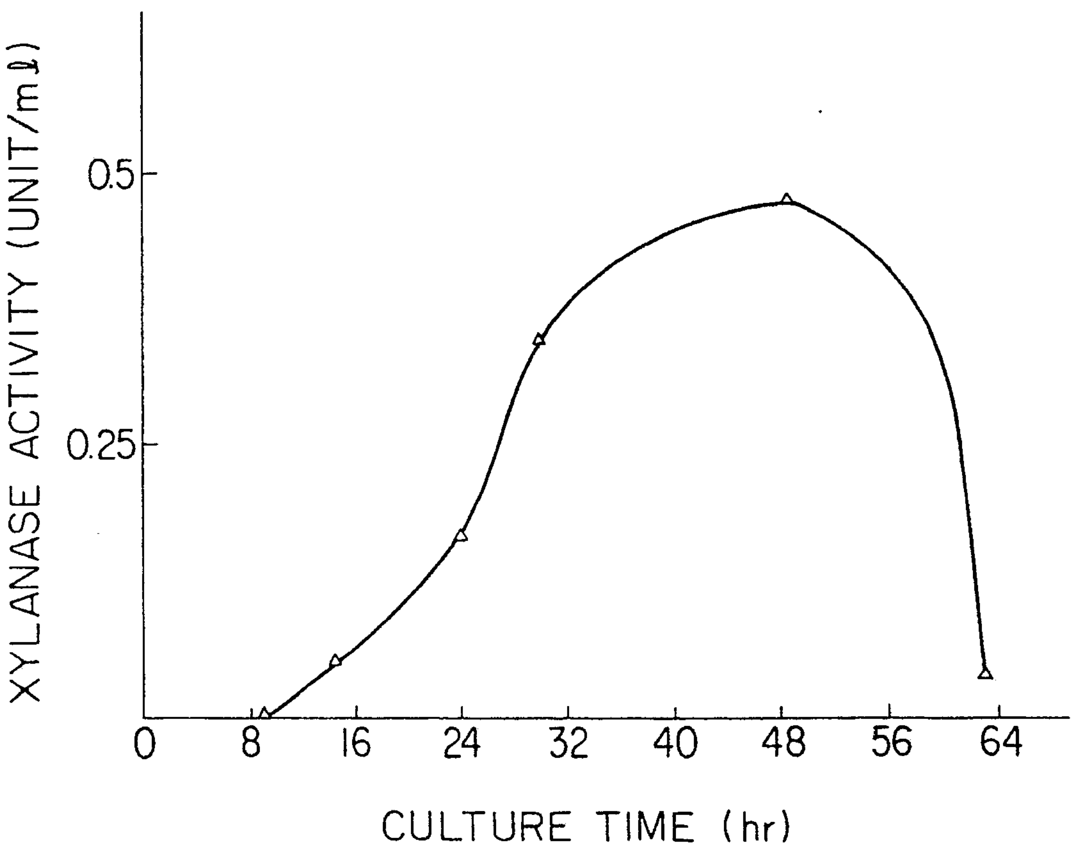
XYLANASE ACTIVITY (UNIT/ml)
0.5
0.25
0
8
16
24
32
40
48
56
64
CULTURE TIME (hr)

FIG. 8

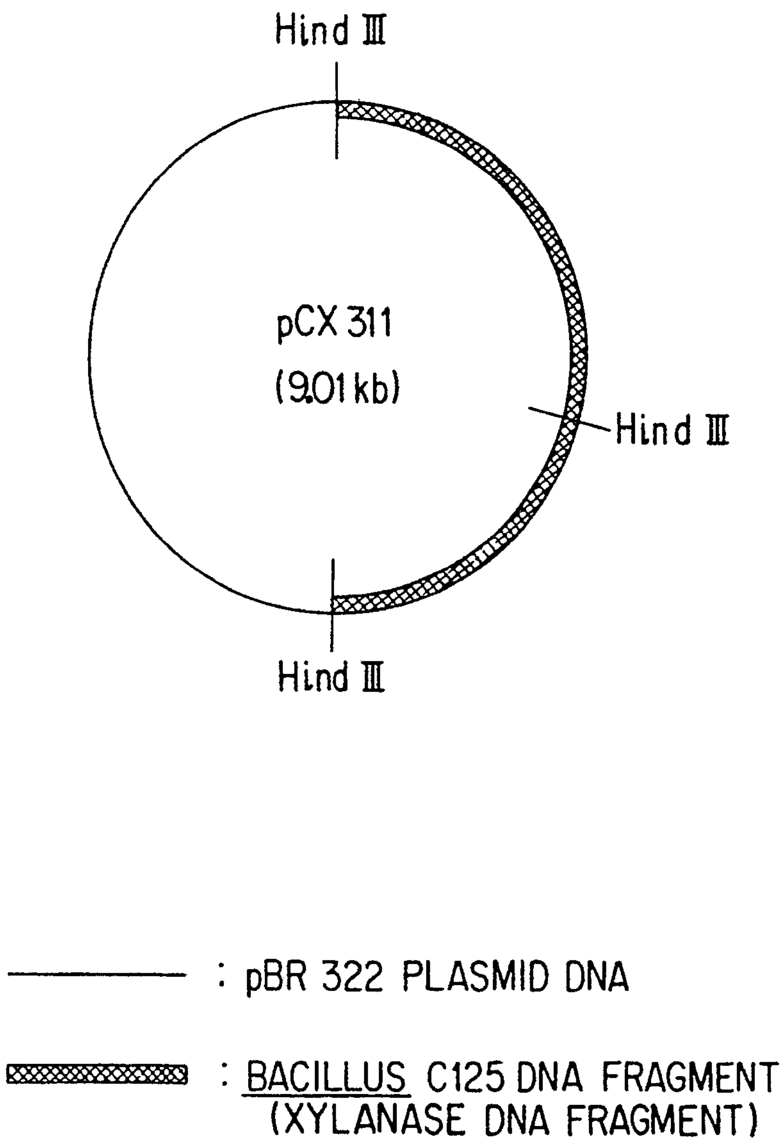
Hind III
pCX 311
(9.01 kb)
Hind III
Hind III
: pBR 322 PLASMID DNA
: BACILLUS C125 DNA FRAGMENT
(XYLANASE DNA FRAGMENT)

# FIG. 9

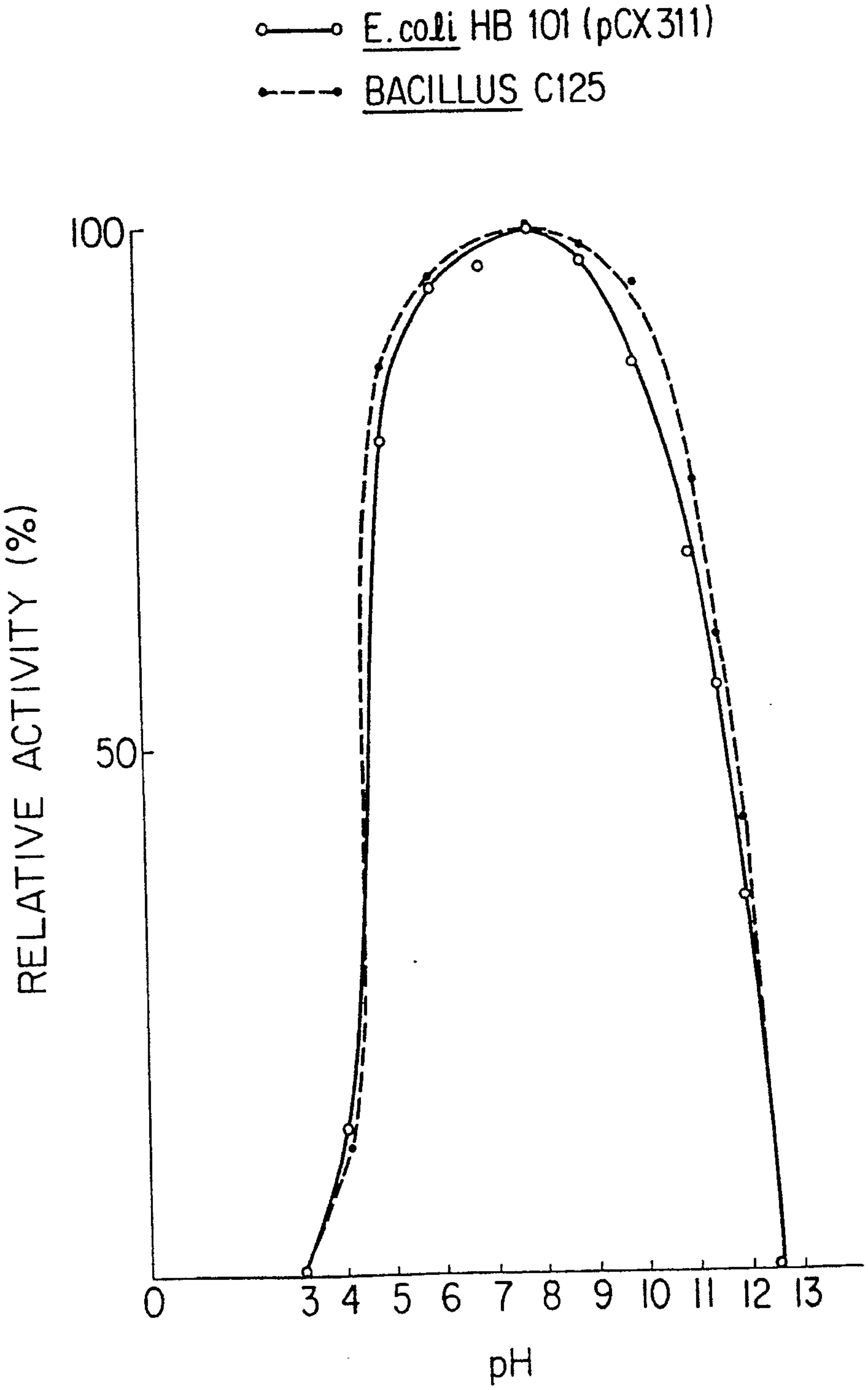
E. coli HB 101 (pCX311)
BACILLUS C125
100
50
RELATIVE ACTIVITY (%)
0
3 4 5 6 7 8 9 10 11 12 13
pH